# EUROPEAN PATENT SPECIFICATION

(11) **EP 1 608 974 B1**
(45) Date of publication and mention of the grant of the patent: **17.10.2007**
(21) Application number: 04724033.8
(22) Date of filing: 29.03.2004
(51) Int. Cl.: G01N 33/543, G01N 33/558

(54) **MULTIPLE-CHANNEL TEST DEVICE, METHOD FOR PRODUCING THE SAME AND USE THEREOF**
MEHRFACHKANAL-TESTVORRICHTUNG, VERFAHREN ZUR HERSTELLUNG DAVON UND VERWENDUNG DAVON
DISPOSITIF DE TEST MULTICANAL, PROCEDE DE PRODUCTION ET UTILISATION DE CE DISPOSITIF

(30) Priority: 28.03.2003 FI 20030463; 19.06.2003 FI 20030921
(43) Date of publication of application: 28.12.2005
(73) Proprietor: Ani Biotech Oy, 00100 Helsinki (FI)
(72) Inventor: NISKANEN, Aimo, FI-02660 Espoo (FI); LAINE, Henri, FI-03600 Karkkila (FI); SARAMÄKI, Mika, FI-03620 Karkkila (FI)
(74) Representative: Lönnqvist, Gunnel Solveig Kristina
(86) International application number: PCT/FI2004/000179
(87) International publication number: WO 2004/086042

(56) References cited:
- WO-A-00/22434
- US-A- 5 110 724
- US-A1- 2003 040 021
- US-B1- 6 171 870
- US-B1- 6 340 588

## Description

### Technical Field of the Invention

The invention relates to an immunodiffusion-based multiple-channel test device that enables the simultaneous or parallel performing of several different analyses. The production and use of the device are also disclosed in the invention.

### Background of the Invention

Methods and test devices based on immunodiffusion are known for example from the following patents and patent applications: US 4,757,002, US 3,990,852, US 4,562,147, and EP 0 250 137. Immunochromatographic methods based on immunodiffusion have been disclosed for example in the following patents and patent applications: EP 0 291 194, EP 0 284 232 (FI 93150), and WO 86/03839.

The above-mentioned test devices based on immunodiffusion and immunochromatography are most often only used for performing one analysis per test device. In many cases, making a definite diagnosis and selecting the suitable method of treatment for a patient suffering from a specific illness or syndrome might require performing several different analyses. If the recognition of the cause of a disease causing the symptoms and the illness or the exclusion of specific illnesses requires performing several different tests, making a diagnosis would become so expensive that usually only one or a very restricted number of analyses are performed, these analyses being randomly chosen based on the judgment of the treating physician or among commonly used tests, in which case other alternatives might be left undefined.

The use of more than one test channel is known from US B1 6,171,870 and US A1 2003/0040021. US B1 6,171,870 relates to a method for producing such channels by applying a water-repellant substance, such as wax on a porous carrier. The treated areas form blocking segments between the channels. In the method described in US A1 2003/0040021 samples moves along treated channels, while untreated areas are left between the channels.

WO 00/22434 discloses a diffusion-based multiple-channel test device suitable for carrying out immunoassays which channels are produced by laser etching.

Immunological test devices having laser etched markings with information concerning the performed test are known from US 6340588. The markings are used to identify the individual test devices and to retrieve the information stored thereon.

Advantages of the present invention over prior art methods and test devices include a decrease in reagent and material consumption, sample volume and freight, as well as improvements in environmental friendliness, shelf-life and user-friendliness. An increment in environmental friendliness is achieved on account of a diminished environmental load caused by used test devices. Due to the savings in reagent consumption and labour costs, the method according to the invention enables the production of the test devices with extremely low costs.

The problems connected with conventional test devices based on immunodiffusion and immunochromatography are overcome with the present invention, the characteristics of which are disclosed in the following claims.

### Summary of the Invention

The immunodiffusion-based test device of the present invention comprisies a porous carrier material and in it, a specific binding reagent (immunoreagent) in the form of a zone or a blot. A detectable label to which a second specific binding reagent (immunoreagent) is coupled, is supplied separately or pre-applied to the porous carrier, and is mobilizable by the sample. A sample application site, optionally provided with a filter, is also situated on the test device. The porous carrier material of the test device is preferably made of nitrocellulose and comprises a network of channels and markings with information concerning the test, which are etched into the porous carrier material with laser treatment. The network of channels comprises two or more channels (1), separated by a treated area (2), one or more specific binding reagents (3) immobilized in them, an optional label site (4) placed in the channel near the sample application site (5), or in the sample application site (5) itself, which is placed in a way that enables an even distribution of the sample into each channel.

The test device of the present invention enables diagnosis of different diseases or syndromes based ona simultaneously or parallelly performed recognition of several syndrome-producing agents or analytes. The test device may be used to simultaneously recognize several allergy-producing agents, myocardial infarction markers, venereal disease-producing agents, blood screening analytes, respiratory infection-producing agents, infectious disease-producing agents and/or cancer markers.

Example of useful specific binding reagents are antibodies, antibody fragments, recombinant antibodies, recombinant antibody fragments, antigens, lectins, receptors and/or ligands. Useful label reagents are for example latex, gold, metal or colouring agent particles, or fluorescent substances.

The invention dislcoses a method for producing the test device, wherein the specific recognizing immunoreagent is immobilized in a porous material, which has been rendered inert with a substance, and wherein the sample application site is optionally provided with a filter and a detectable label to which a specific binding reagent is bound. A channel network of a desired shape and size and markings with information concerning the test are formed on the porous material by etching the porous material with laser. The network comprises two or more channels (1) and a treated area (2); and one or more recognizing specific binding reagents are immobilized in each channel (1) as a zone or blot (3).

Useful substance by which the porous material is rendered inert is a mixture comprising natural or synthetic polymers, such as albumin and casein or PEG (polyethylene glycol) and PVA (polyvinyl alcohol), nonionic detergents such as hexane sulphonic acid and TRITON-X-100, BRIJ, and preservatives such as sugar, for example sucrose and trehalose, or their derivatives.

The storability of the test device is highly improved by drying and keeping the the test device in a relative humidity of not over 8 % and hermetically packed after application of the reagents.

The multichanneled test device is useful for performing simultaneously or parallelly several assays, which enable the diagnoses of a target disease and/or syndrome, which may be diffiult to diagnose using only one marker. The sample is applied to the application site (5) of the test device containing a label mobilizable by the analyzable sample and a second specific binding reagent coupled to it, from which site the sample and the reagents migrate evenly into the channels of the channel network formed by the laser treatment, where they either react or do not react with the specific binding reagent in its immobilization site (3), from which site the positive or negative results are directly readable.

The present invention is also related to a test device for simultaneous or parallel performing of several assays for recognition of the target disease and/or syndrome. The the sample is applied to an application site (5) of the test device, from which it migrates into a channel where it is mixed to and reacts with the label mobilizable by the sample and the second specific binding reagent bound to it, after which the sample and reagents migrate evenly into the channels of the channel network formed by a laser treatment, where they either react or do not react with the specific binding reagent in its immobilization point (3), from where the positive or negative results are directly readable.

The present invention is also related to the use of the test device for simultaneous or parallel performing of several assays for recognition of a target disease and/or syndrome. The sample is mixed with a separate label having a second specific binding reagent coupled to it and the mixture is applied to an application point (5) of the test device, from which the sample and the reagents migrate evenly into the channels of the channel network formed by a laser treatment, where they either react or do not react with the specific binding reagent in its immobilization point (3), from where the positive or negative results are directly readable.

### Brief Description of the Drawings

Figure 1 shows a network of eight channels, which is one of the preferred test devices for performing up to eight different assays. The label site 4 simultaneously functions as the sample application point 5 and the channels 1 separated by treated areas 2 contain the specific binder as the zone 3.
Figure 1a shows an advanced embodiment of the multiple-channel test device for performing up to eight different assays according to Figure 1. The label point 4 simultaneously functions as the sample application point 5 and the channels 1 separated by treated area 2 contain specific binder as the zone 3. The device is marked with information concerning the tests and the manufacturer.
Figure 2 shows a test device that can be used for performing six different assays and their control reactions or, optionally, up to twelve different assays by six different labels. One of the channels 1 branches into two channels 1a, 1b that are separated by treated area 2. The sample application point is located in the middle of the channel network and each channel 1 comprises a label point 4 and one or more binder zones 3.
Figure 3 shows a test device that can be used for performing four different assays and their control reactions, or optionally, up to eight different assays by four different labels.
Figure 4 shows a test device that can be used for performing four different assays and their control reactions, or optionally, up to eight different assays by four different labels.
Figure 5 shows a test device that can be used for performing up to six different assays, or optionallythree assays and their control reactions.
Figure 6 shows a test device that can be used for performing twelve different assays by four different labels.
Figure 7 shows a network of eight channels, which is one preferred test device for performing up to twenty-four different assays. Each channel 1 comprises three binder zones 3.
Figure 8 shows a test device that can be used for simultaneously performing sixteen different assays.
Figure 9 shows a test device that can be used for performing twenty different assays by five different labels.
Figure 10 shows a test device that can be used for performing two assays and a control reaction. The device is marked with information concerning the tests and the manufacturer.
Figure 11 shows a test device that can be used for performing two assays and a control reaction. The device is marked with information concerning the tests and the manufacturer.
Figure 12 shows a test device produced by laser etching that can be used for performing three parallel assays or, optionally, three individual assays and their control reactions. The device is marked with information concerning the tests and the manufacturer.

### Detailed Description of the Invention

The object of the invention is an immunodiffusion-based multiple-channel test device comprising porous carrier material and in this material, one or more specific binding reagents (immunoreagents) in the form of zone(s) or blot(s) and a sample application point that is optionally equipped with a filter for example for the removal of blood cells, and which optionally comprises a label mobilizable by the analyzable sample, coated with a second specific binding reagent (immunoreagent). The label is on the test device or it is added to the sample.

The test device according to the invention is characterized in that it comprises a channel network and markings with information concerning the test produced by etching a network on a porous carrier material for example by laser. The channels of the test device have been provided with one or more identical or different specific binding agents that are selected from different marker groups, which can be used together and are required for diagnosing a specific syndrome. Thereby, the diagnosis is enabled by the simultaneous performing of several assays for the recognition of a specific target illness and/or syndrome. The sample application site, which can be in the form of a dot or a blot or a line or a zone, is located for example in the middle or the other end of the strip in a way that enables an even distribution of the sample into each of the channels. If the same specific binding reagent is used in the channels in different concentrations, a semi-quantitative result can be obtained.

The test device according to the invention comprises several channels that can be used for the simultaneous or parallel determination of several analytes. In the test device, it is possible to optionally group together reagents that recognize disease-producing agents associated with various syndromes, for example allergens, myocardial infarction markers, suitable reagents for the recognition of venereal diseases and for blood screening, markers that recognize respiratory infection producing agents, IgG, IgA and IgM antibodies, markers that recognize other infectious disease producing agents as well as various cancer markers, for their simultaneous or parallel determination.

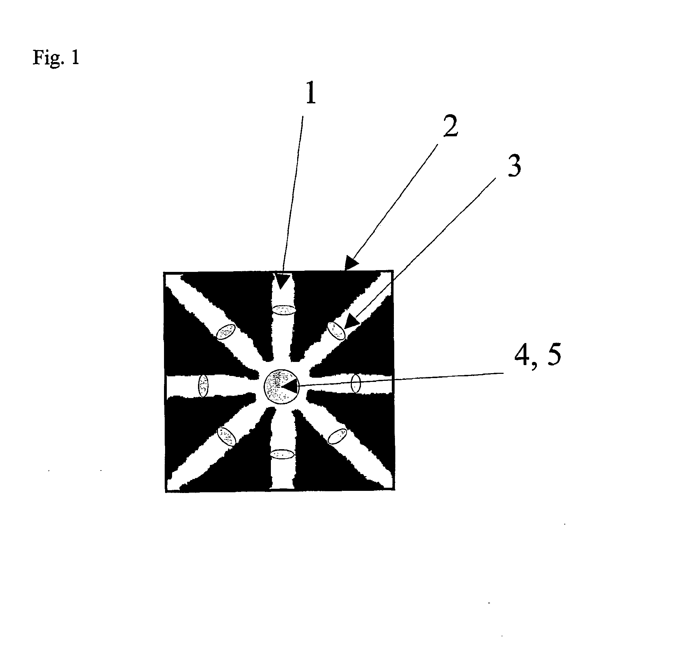

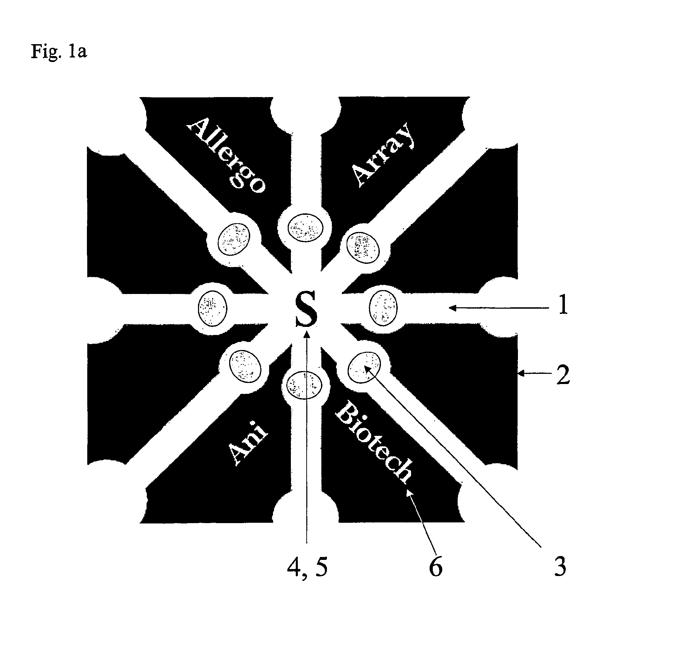

An additional object of the invention is a method of producing said test device. The production method is characterized by that a multiple-channel channel network of a desired shape and size and marking with information concerning the test (see Figs. 1-12) is etched on a porous material by laser-techniques, which make a certain shape on the substrate. Different recognizing specific binding reagents are permanently attached, i.e. immobilized, in the porous substance of each of the channels. The analysis results for the test reactions are readable from these points.

After this, the porous material is treated with a substance that renders the free reactive sites inert, i.e. they do not react in an undesirable way, for example by slowing down or preventing the analytes or labels from migrating into the permanently immobilized reagent zone or blot. The detectable, optionally visible label, on which a second specific binding reagent is bound, is applied to the sample application point or to the test channel on a pre-determined place. The label to which the binding reagent is bound can also be added to the sample, in which case the label is transferred into the device as sample is added to the sample application point.

In addition, the use of the test device for the simultaneous or parallel performing of several assays to recognize a certain illness and/or syndrome is described in the invention. The branched channels may also be used for controlling the appropriate functioning of the device, using one of the channels as a test channel and the other as a functionality or a control channel. A separate control channel indicating the functionality of the device and the reagents can also be added to the test device.

The multiple-channel test device according to the invention as well as its use in diagnostic methods differ from the prior art test devices disclosed in the above-mentioned patents and patent applications in that one or more different or identical parallel tests, preferably 2-40, more preferably 4-30, and most preferably 8-24, parallel tests can be performed by using an extremely small object produced from a porous material.
The use of laser for producing the channel network and markings with information concerning the test is a preferred method for the manufacture of the test devices according to the invention. The machine used in this production method is inexpensive, and operating costs mainly consist of the amount of electricity used. As compared to the use of a water-repellent substance such as hot wax, by laser manufacturing the use of chemicals during the manufacturing process can be avoided. By using laser, a precise etching result (printing) can be obtained, and because of the preciseness of the printing (impression), the testing devices may, if desired, be marked in connection with the manufacturing process, thereby diminishing the risk of mixing up the devices.

Large amounts of tests may be manufactured by etching single channels, parallel channels or channel networks on a roll and adding the required reagents and treatments on the same production line and finally separating the tests from each other by a desired manner into single tests for one or several assays or into test combinations of several parallel tests.

In addition, the test device can be used for analyzing small volumes of patient samples, such as urine, blood, plasma, serum, saliva, tissue fluids, faeces, environmental samples, etc. The small sample volume, even as small as 1,0-50 µl, preferably 2,0-40 µl, more preferably 4,0-20 µl, or most preferably 5,0-10,0 µl per test device being sufficient, advantages are gained especially in regard to performing assays on samples collected from small children or finger-tip blood samples.

### Producing the test device

The production of the test device comprises six main steps. Forming the channel network and markings with information concerning the test applying and immobilizing the specific reagent, rendering the porous channel inert, applying the label coated with a second specific reagent, producing the sample application point and stabilizing the test device and ensuring its storing properties. Between the main steps mentioned, the test strip may be dried. If the label is added to the sample, the step of applying the label to the test device can be omitted.

In the test device according to the invention several different or identical parallel tests (Figs. 1-12) can be performed on a very compact item of a porous material by channelling the test device.

### Producing the channels

The channelling of the reagents and the samples can be achieved by treating a porous, water-transmitting material, for example nitrocellulose, polysulphonate, nylon or paper, with a water-repellent or partly water-repellent substance, such as wax, polyolefines, polyacrylamide paints or mixtures thereof. The application of water-repellent materials on the porous material is performed at their melting temperature, preferably at a temperature between 50 °C and 80 °C, advantageously by using a printing, brushing or spraying technique, thus forming on the porous material a water-repellent figure that is of a desired shape and that encloses a channel network of several channels separated from each other by the treated area, along which channels the sample migrates due to the effect of capillary action and diffusion. After the application, the substrate is immediately cooled down to room temperature. It is possible to apply the reagents into the channel network in a way that maintains their reactivity.

Naturally, the channels can also be produced by stamping out a suitable-size piece of carrier material to form a channel network of a desired shape and size.

However, according to the present invention the channelling is preferably performed by treating a porous and water-transmitting material, such as nitrocellulose, with laser. By using suitable laser power, nitrocellulose can be etched away from such areas of the nitrocellulose substrate into which fluid flow is not wanted, leaving the treated areas covered only by the plastic film (Mylar film) underlying the cellulose. Figures of different shapes (Figs. 1-12) are formed by the channels containing nitrocellulose or some other porous material and the channel edges or treated areas that extend all the way to the substrate edges and have been laser-etched by using a suitable pre-programmed computer program, using 10-90 % of the maximum output power of the device, preferably 10-80 %, more preferably 12-60 %, most preferably 15-40 %, an etching speed that preferably ranges from 100 mm/s to 1 500 mm/s, more preferably from 400 mm/s to 1 000 mm/s, and a resolution that ranges from 50/1 000 to 1 000/1 000, preferably from 100/1 000 to 800/1 000, more preferably from 150/1 000 to 500/1 000, most preferably from 200/1 000 to 300/1 000.

The treated area separating the channels may extend all the way to the edges of the test device or, optionally, untreated area may be left on figure edges. The test device is marked with information concerning the manufacturer or the tests by etching, for example by laser treatment.

### The application of the reagents into the carrier

It is possible to apply and, if needed, to immobilize the various reagents required for performing the tests in the channel network. The reagents may be used in very small volumes. The required reagents comprise at least two specific binding reagents, of which one is immobilized and the other bound to the label mobilizable by the sample solution, and at least one detectable label.

### Specific binding reagents

Suitable substances include various binding reagents, specific immunochemical reagents such as antibodies, antibody fragments, recombinant antibodies, recombinant antibody fragments, antigens, but also other ligands such as receptors, lectins, biotin, avidin, etc. Especially suitable are monoclonal and polyclonal antibodies, antigens and fragments thereof. As regards allergy testing, suitable allergens include extracts prepared from the pollen of trees, grass or weed, extracts prepared from spores of moulds, acarids, household dust, animal skin epithelium, insects, latex, parasites, drugs or foodstuff, among others.

Binding reagents may be immobilized or chemically or physically attached by a known method to the structure of the porous carrier on the desired points, and a binding reagent for a control reaction may also optionally be immobilized or chemically or physically attached to the same or a branched channel.

### Rendering the porous substance inert

After producing the channel network and applying the specific binding reagent, the porous carrier substance is rendered inert by using a so-called blocking agent, whereby the free reactive sites of the carrier substance and the non-specific binding sites of the specific binding reagents are eliminated by a suitable mixture containing natural and/or synthetic polymers such as albumin or casein and/or PEG (polyethylene glycol) and PVA (polyvinyl alcohol), nonionic detergents such as hexane sulphonic acid and TRITON-X-100, BRIJ, and preservatives such as sugar, for example sucrose and trehalose, or their derivatives. After this, the test device is dried.

If desired, the blocking agent may be added only after the production of the test device, in connection with the application of the sample.

### Producing the label point

Suitable labelling agents include various plastic or metallic microparticles mobilizable by sample flow, such as latex, gold, liposomes, colouring agents, fluorophors, fluorochromes or other such particles of a metallic substance or a colouring agent that are able to bind the analytes and bind to the specific binders such as antibodies, receptors, lectin or other ligands. Also fluorescent particles, fluorescent colouring agents or superparamagnetic particles may function as labels.

The label may be immobilized in the porous carrier, to the sample application point of the test device or to an another point separate from the sample application point. The labelling agent may also be added to the analyzable sample before it is applied on the test strip.

### Producing the sample application point

The sample application point may optionally be provided with a filter by placing on the sample application point a filter that prevents for example blood cells from being carried into the channels. The sample application point may be provided with a label to which a second specific binding reagent has been bound.

### Stabilizing the test device and ensuring its storing properties

The test device can be made storable by treating it for example by drying to give it a relative humidity of below 8 %, after which the test device is hermetically packed and stored dry in order to maintain the relative humidity of the test device below 8 %. Thus, the device maintains its functionality for as long as 24-36 months, without any substantial decrease in functionality, including sensitivity or specificity.

When necessary, the test device may be placed inside a casing made of paperboard or plastic, which can, where needed, be equipped with instructions describing the operation and use of the test device.

### The structure of the test device

The structure of the test device and its possible variations are described in Figures 1-12.

The test device comprises of several, preferably 2-10, more preferably 3-8, channels 1 which may on the branch point further divide into several, preferably 2-5 (1a, 1b...), more preferably 2-3, branched channels. A treated area 2 separates the channels 1 from each other and directs the migration of the sample in the test device.

The channels 1 or branched channels (1a, 1b...) contain one or more binding reagents as a binder zone or blot 3. In addition, the test device comprises one or more label points 4, which may optionally be combined with the sample application point 5. The sample application point 5 may optionally be provided with a filter.

Figure 1 shows a test device wherein a nitrocellulose film of a desired size, e.g. 25 x 25 mm, has been divided into eight identical channels 1. The channels have been prepared by printing a figure on a porous material, for example on nitrocellulose film, and transferring hot, melted, about 60 °C polyolefin on the treated water-repellent zones 2 of the nitrocellulose film with a stamping device. Each channel contains a specific binder 3. The label point 4 in the middle of the test device contains a second label reagent directed against the analyte to be determined. The sample application point 5 simultaneously functions as the label point 4 of the test device. The test device according to Figure 1 is characterized in that each of the channels may contain a different specific binder (e.g. an allergen) and that a particle label (e.g. anti-human IgE) common to each of the binders is located in the middle of the channels.

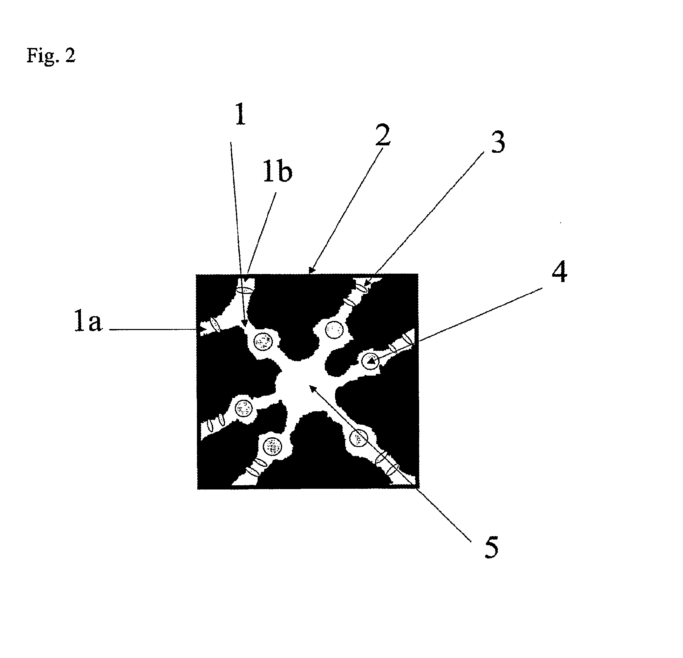

Figure 1a shows a test device wherein the nitrocellulose film has been divided into eight identical channels 1. The channels have been produced by laser-etching a figure on a porous material such as nitrocellulose film in a way that a treated area 2 is left between the channels, on which area markings concerning the tests and the manufacturer have been added during the laser treatment. Each of the channels contains a specific binder 3. Located in the middle of the test device, the label point 4 contains a second label reagent against the analyte to be determined. The sample application point 5 simultaneously functions as the label point 4 of the test device. The test device according to Figure 1 is characterized in that each of the channels may contain a different specific binder (e.g. an allergen), and that a particle label (e.g. anti-human IgE) common to each of the binders is located in the middle of the channels. Figure 2 shows a test device wherein one of the channels 1 comprises branched channels 1a and 1b containing the specific binder in the binder zone 3. Other channels 1 comprise two binder zones 3, of which the other may be a control zone. All channels 1 contain a separate label point 4. At its centre, the device comprises a separate sample application point 5.

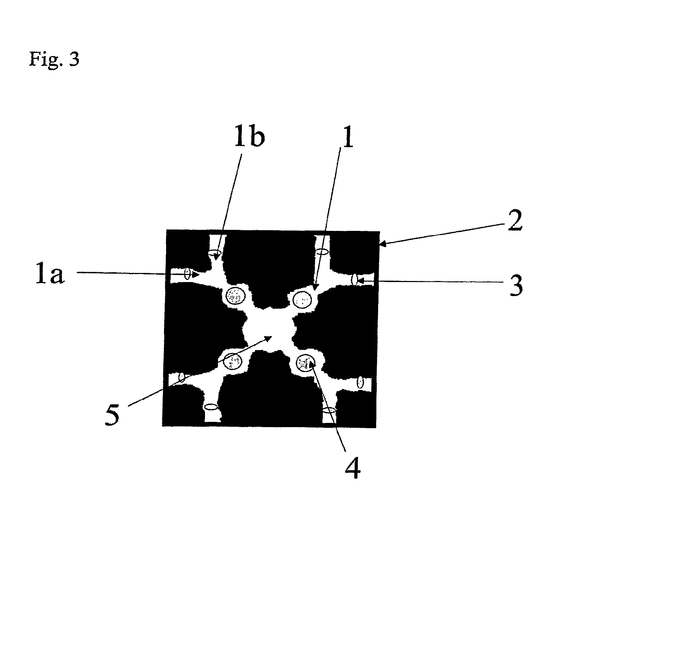

Figure 3 shows a test device wherein four identical or non-identical channels 1 comprise the branched channels 1a, 1b that contain specific binders in the binder zone 3 and downstream from the branch points of the channels four separate particle label points 4 that have been coated with specific binders. The device comprises a separate sample application point 5.

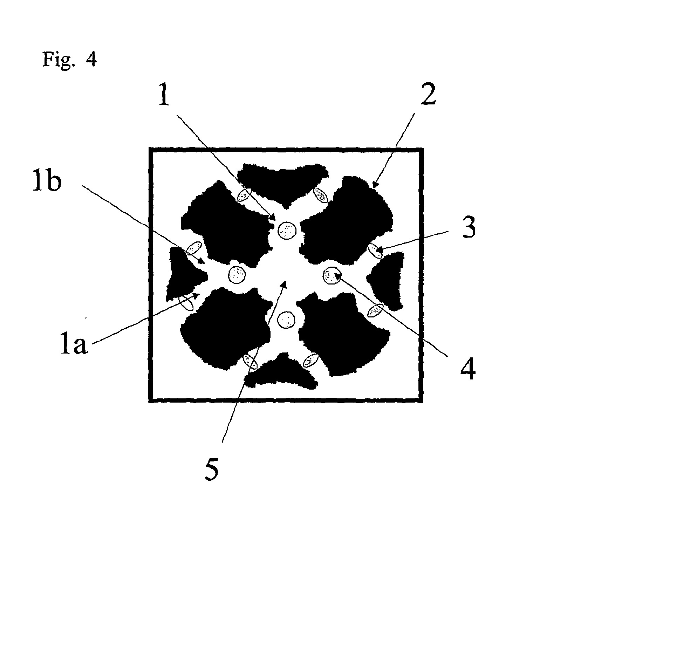

Figure 4 shows a test device with four identical or non-identical channels 1 that branch into channels 1a, 1b, which in turn contain specific binders in the binder zone 3 and downstream from the branch points of the channels four separate particle label points 4 coated with specific binders. The device comprises a separate sample application point 5.

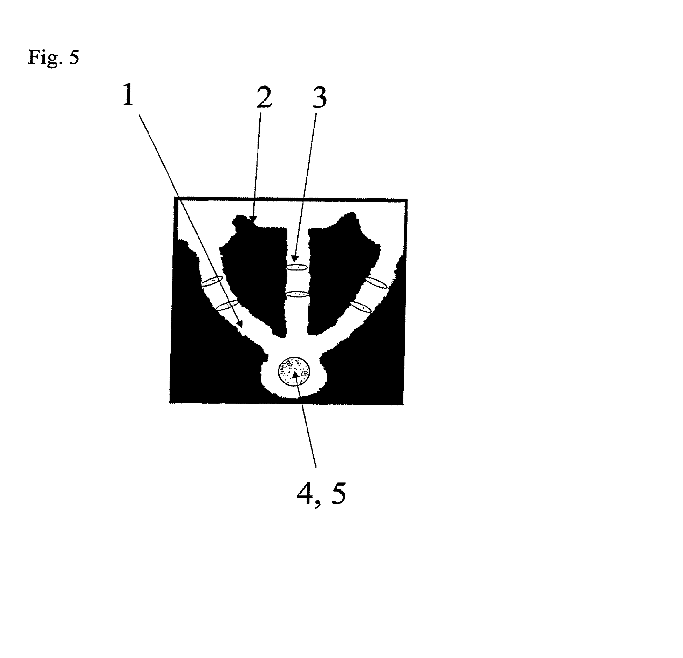

Figure 5 shows a test device wherein the sample application point is located at one end of the test device or strip. The device comprises three channels 1 with two binder zones 3 located into each of them. The label point 4 is the same as the sample application point 5.

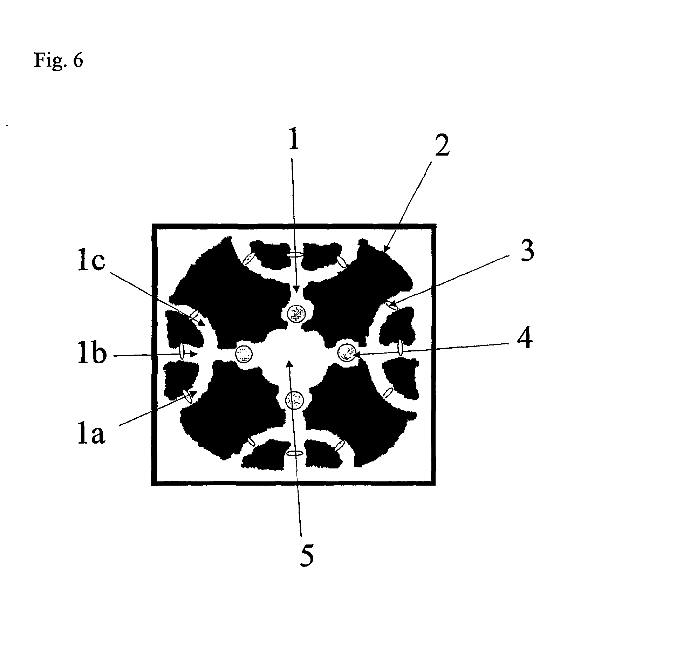

Figure 6 shows a test device that comprises up to twelve different binders in twelve separate binder zones 3 in four different channels 1 that further branch into three channels 1a, 1b and 1c. Furthermore, the device comprises four separate label points 4. The test device comprises a separate sample application point 5.

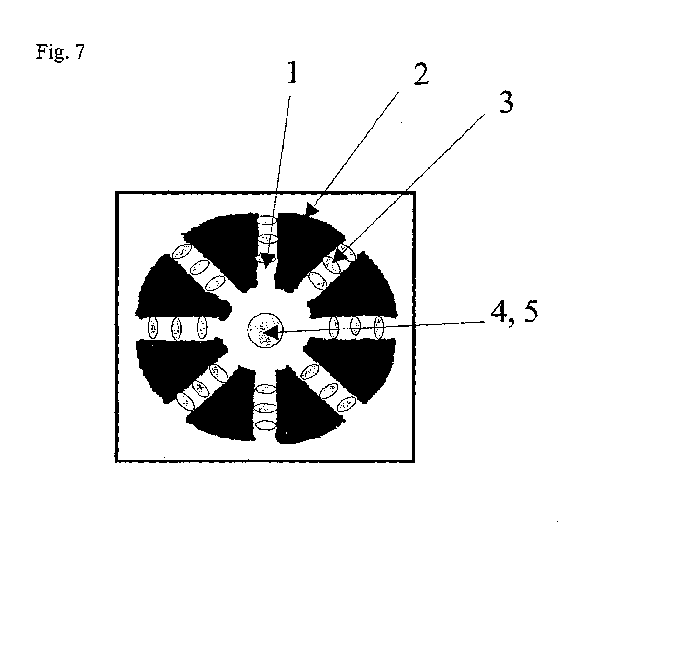

Figure 7 shows a test device comprising eight identical channels 1 and between them treated areas 2. Each channel 1 contains three specific binders, optionally against different analytes, in three separate binder zones 3. The label point 4 in the middle of the test device contains another label reagent against the analyte to be determined.

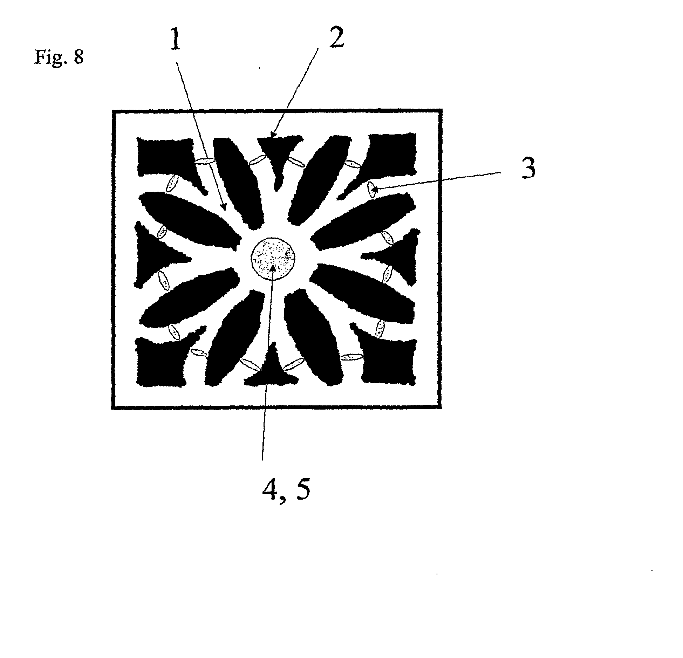

Figure 8 shows a test device comprising eight channels 1, each branching into two channels 1a and 1b and containing a binder zone 3. The device comprises a combined label and sample application point 4, 5.

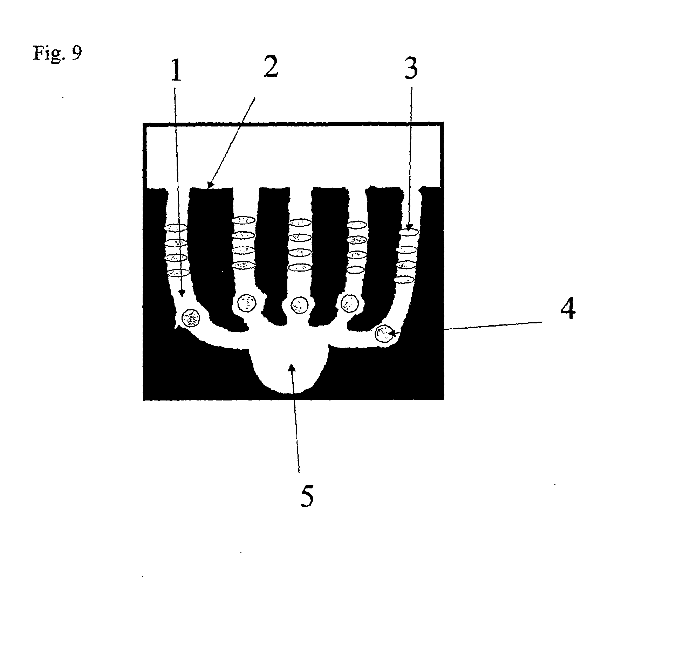

Figure 9 shows a test device comprising five channels 1, each channel comprising four binder zones 3. The device comprises four separate label points 4 and a sample application point 5.

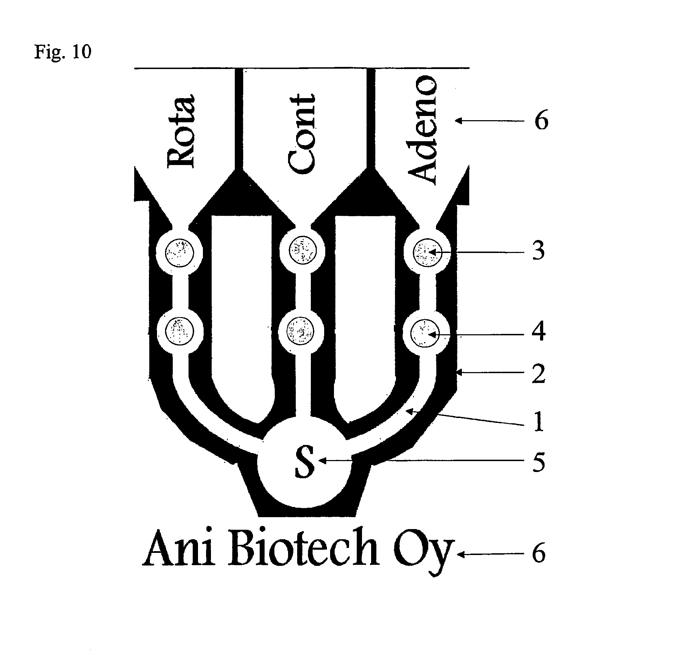

Figure 10 shows a test device for detecting virus antigens, in which the sample application point 5 is placed at one of the ends of the test device or strip. The device comprises three channels 1, each of them containing a binder zone and a separated label point 4. The device is marked with information concerning the test and the manufacturer.

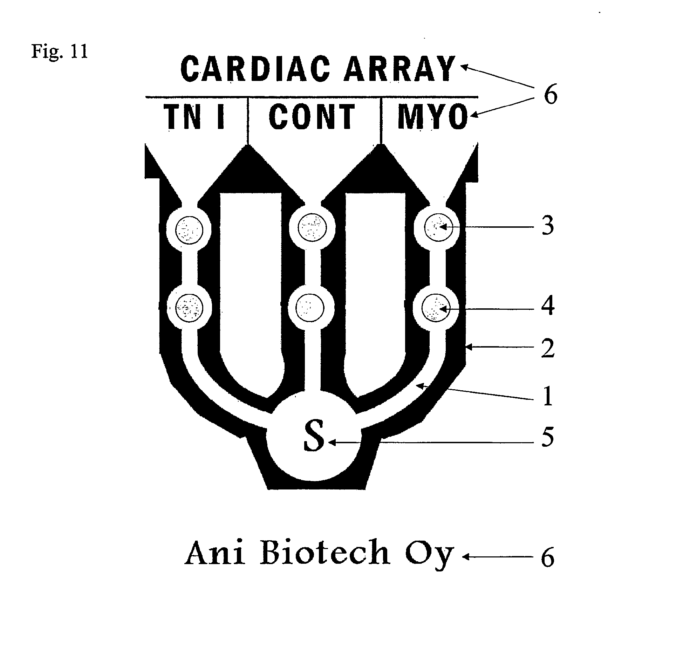

Figure 11 shows a test device for myocardial infarct detection, in which the sample application point 5 is placed at one of the ends of the test device or strip. The device comprises three channels 1, each of them containing a binder zone 3 and a separated label point 4. The device is marked with information concerning the test and the manufacturer.

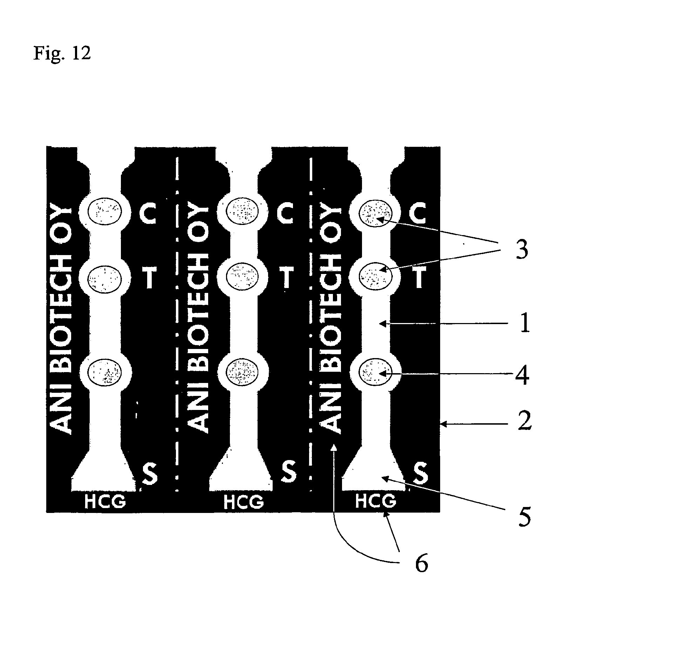

Figure 12 shows three test devices, in which the sample application point 5 is placed at one of the ends of the test device. The devices comprise a label point 4 and binder zones 3, of which the other is a control point.

The form and size of the above described test devices are only to be seen as examples of those that the process according to the present invention enables to produce.

### Use of the test device to perform a simultaneous or parallel assay

The present invention is based on fluid flow in a porous material such as nitrocellulose taking place practically in every direction at the same speed due to diffusion and capillary action as is well known. The said radial or lateral flow enables the movement of the sample, that contains the analyzable analytes and test reagents, into several channels that simultaneously serve as different tests, as far as the channelization of the porous material has been taken care of in a secure manner. The sample applied to the test device reacts with the label added to the sample or with one or more labels applied to the device and this complex in turn reacts with the immobilized binders placed further in different channels of the test device. Also immunological reagents can be immobilized without interfering with their functionality. The application of the sample to the test device is performed by applying a small amount of the sample or its dilution to the sample application point 5, from where it moves to the label point(s) 4 and further along the channels into the test zones 3, where the reaction between the specific binder of the label, the analyte in the sample and the other specific binder bound to the solid carrier takes place. This reaction is rendered visible to the naked eye if the label has been a coloured particle or it can be read under UV-light by naked eye as a light emitting dot, blot, line or other figure or it can be read and interpreted with a suitable device, photometer, fluorometer or a device that measures the changes of the magnetic field.

In another embodiment the label point 4 is placed in the sample application point 5, in which case the analyte in the sample and the specific binder of the label form a complex before the sample flows along the channels.

In a further embodiment the application of the label to the sample is performed before adding the sample to the test device.

The test device is especially useful in allergy testing, where a relatively large amount of serum is now required to define the allergy specific antibodies of class IgE. When applying the present invention, the sensibilization of a patient to up to 24 or more different allergens can be detected from a very small amount of sample, even only 10-40 µl of serum, plasma or whole blood, simultaneously with the same test device by dosing several different allergens to each channel of the test device.

In one preferred embodiment of the present invention several different labels are attached to the test device. Thus, one small sample mobilizes the labels that are optionally different from each other and placed in different channels. A test device like this is well suited for immunoglobulin specific antibody assays against different disease producing agents.

### Examples of applying the test

The following embodiments are to be seen as examples and it should be understood that other possible applications of the present invention are obvious to a person skilled in the art.

### Example 1. Forming a channel network by laser

The figure shown in Figure 1a is prepared on a porous nitrocellulose carrier by etching nitrocellulose from the areas to which fluid flow is not wanted by using a Domino DGM-1 "High Resolution Laser Marker" device. A network that comprises eight channels 1 separated by treated areas 2 is formed on the test device. To avoid the risk of the devices getting mixed up, markings concerning the product and the manufacturer are formed by leaving nitrocellulose unetched.

The desired figure pre-programmed into the computer programme is etched by using a marking speed of 700 mm/sec of 10-2500 mm/sec and 20 % of the maximum output power of 20W Laser (85-132 V/170-260 V, I Phase input and 20W output) and a resolution of 250/1000.

The thus produced test device matrix with a channel network is used according to the examples below for performing several assays simultaneously.

### Example 2. A test device for allergy testing

Specific analyzable allergens are dosed as solutions (in a concentration of 1-5 mg/ml) to the channels 1 of the network of the test device produced according to Example 1, so that they form insoluble zones 3 as they attach to the nitrocellulose of the sample application point. Different allergens are applied to each channel (extracts prepared from the pollen of trees, grass or weeds, extracts prepared from mould spores, acarids, household dust, animal skin epithelium, insects, latex, parasites, drugs or foodstuff). After this, the reactive nitrocellulose of channels 1 is blocked, i.e. rendered inert, by using a solution containing bovine serum albumin (BSA) (0,1-5,0 %), hexane sulphonic acid and trehalose (1,0-3,0 %). The solution (10-100 µl) is applied into the middle of the test device, after which it migrates into each channel due to diffusion and capillary action and binds, i.e. blocks, the free reactive points of the nitrocellulose.

After this, the test device is dried at room temperature until its relative humidity is below 8 %.

After drying, 1,0-10,0 µl of an aqueous solution containing coloured latex particles coated with anti-IgE (0,2-1,0 %), the mixture further containing 0,1-1,0 % of BSA, 0,01-0,05 % of Tween 20 and 0,5-1,5 % of trehalose is manually applied into the middle of the test device. The particle sol is dried into the middle of the test device.

When using the test, 10-50 µl of a serum or its dilution from a person suffering from an allergy is applied into the middle of the test device to the sample application point 5. The sample dissolves the label in the middle. The anti-IgE antibodies of the label react with the allergy specific IgE molecules and diffuse into the test channels due to capillary action.

If the analyzable sample contains specific IgE antibodies against one or more allergens in the zones 3 of the test device, a coloured zone is formed in the reaction channel that contains the antibody of the allergen in question.

### Example 3. A test device for allergy testing

Using a suitable stamp, a figure formed as shown in Figure 1 is printed on the porous nitrocellulose carrier, wherein a channel network is formed, by transferring coloured about 60 °C polyolefin on the porous carrier so that it blocks the pores of the carrier in the desired areas and a network that contains eight channels 1 separated by treatedareas 2 is formed on the test device.

The specific allergens to be tested (in a concentration of 1-5 mg/ml) are applied to the channels of this network so that they form insoluble zones 3 attaching to the nitrocellulose of the sample application point. Different allergens are applied to each channel (extracts prepared from the pollen of trees, grass or weeds, extracts prepared from mould spores, acarids, household dust, animal skin epithelium, insects, latex, parasites, drugs or foodstuff). After this, the reactive nitrocellulose of the channels 1 is blocked, i.e. rendered inert, by a solution containing bovine serum albumin (BSA) (0,1-5,0 %), hexane sulphonic acid and trehalose (1,0 -3,0 %). The solution (10-100 µl) is applied into the middle of the test device, after which it migrates into each channel due to diffusion and capillary action and binds, i.e. blocks, the free reactive points of the nitrocellulose.

After this, the test device is dried at room temperature until its relative humidity is below 8 %. After drying, 1,0-10,0 µl of an aqueous solution containing coloured latex particles coated with anti-IgE (0,2-1,0 %), where the mixture further contains BSA 0,1-1,0 %, Tween 20 0,01-0,05 % and trehalose 0,5-1,5 %, is applied in the middle of the test device. The particle sol is dried into the middle of the test device.

When using the test, 10-50 µl of a serum or its dilution from a person suffering from an allergy is applied into the middle of the test device to the sample application point. The sample dissolves the label placed in the middle. The anti-IgE antibodies of the sample react with the allergy specific IgE molecules and diffuse into the test channels due to capillary action.

If the analyzable sample includes specific IgE antibodies against one or more allergens placed in the zones 3 of the test device, a coloured zone is formed in the reaction channel that contains the antibody of the allergen in question.

### Example 4. A test device for venereal disease testing and blood screening

Example 4 describes the application of the present invention to venereal disease testing where both antibodies and viral- and bacterial antigens, are determined simultaneously from patient samples.

The test device shown in Figure 2, in which a multiple-channel network is produced as described in Example 1, is further prepared so that the first channel 1 that branches into two parts forms test channels for HIV1 and HIV2 antibody tests. The polypeptide or recombinant antigens typical of the virus in question, are placed in these channels as lines (0,1-0,5 µl), in a concentration of 0,5-5,0 mg/ml. A coloured label point 4, produced by coating for example gold particles with a third polypeptide or recombinant antigen that recognizes HIV1 and HIV2 viruses, is placed in the channel 1.

The second channel 1 of the same test device is used to detect a HIV virus antigen by placing for example a line or blot of a monoclonal antibody (0,5-5,0 mg/ml) produced against the p24-antigen of the HIV virus into that channel and a so-called control reagent zone comprising a monoclonal antibody against the same label antibody in question in the same unbranched channel 1.

The third channel 1 is used to detect antibodies of the leukaemia virus (HTLV-1/2) by producing a test line 3 and a control line 3 in the way described above of a recombinant antigen typical of this virus and placing the required label, which is produced by coating gold particles with another recombinant antigen typical of the HTLV 1/2 virus in the label point 4 of the same channel.

In a similar manner, the reagent zones 3 prepared from specific recombinant antigens suitable for detecting the *Treponema pallidum*-bacterium antibodies are placed in the fourth channel 1.

In the fifth channel 1, a test system detecting the surface antigen of Hepatitis B virus is placed using two specific antibodies produced against the surface antigen, one in the test zone 3 and the other in the label point 4.

The required reagents to detect Hepatitis C virus are placed in the sixth channel 1. A recombinant antigen typical of HCV is placed in the test line 3 and the label point 4 is prepared by coating gold particles with anti-human IgG.

All channels 1 of the test device are treated with a so-called blocking solution containing albumin (BSA) (0,1-5,0 %), TRITON-X-100, BRI and sucrose by dosing a sufficient amount of this to the sample application point 5 of the test device, from where it diffuses into each channel and fills the reactive points of the nitrocellulose. The drying of the test device is performed in a vacuum cabinet to accelerate drying.

After this, the required above-mentioned labels that are characteristic to each test are dosed to the predetermined label points 4 of the test device by using a suitable automatic dispenser device. The drying is performed as described in the above-mentioned examples.

When analyzing patient samples, 10-50 µl of serum, plasma or whole blood is applied into the middle of the test device and the results can be read after a reaction time of 1-10 minutes. In case of a positive result, a coloured line or blot appears in the test zone and a second coloured zone in the control zones of those channels or branched channels that have one.

### Example 5. A test device for venereal disease testing and blood screening

Example 5 describes the application of the present invention to venereal disease testing, where both antibodies and virus- and bacterium antigens are simultaneously determined from patient samples.

To detect a HIV virus antigen, the test device shown in Figure 3, in which the channelization is made as described in Example 1, is produced by placing a blot prepared from a polyclonal antibody (0,5-5,0 mg/ml) produced against p24 antigen of the HIV virus into the first channel 1, and a so-called control reagent zone that is a polyclonal antibody against the label antibody in question into the same unbranched channel.

In a similar manner, the reagent zones 3 prepared from the specific recombinant antigens suitable for detecting the antibodies of *Treponema pallidum*-bacterium are placed in the second channel 1.

In the third channel, a test system that detects the surface antigen of Hepatitis B virus is placed using two specific antibodies produced against the surface antigen, one in the test zone 3 and the other in the label point 4.

The required reagents for the detection of the Hepatitis C virus are placed in the fourth channel 1. A recombinant antigen typical of HCV is placed in the test line 3 and the label point 4 is prepared by coating gold particles with anti-human IgG. All channels 1 of the test device are treated with a so-called blocking solution containing casein (0,1-5,0 %), hexane sulfonic acid and trehalose (1,0-3,0 %) by applying a sufficient amount of this to the sample application point 5, from where it diffuses into each channel and fills the reactive zones of the nitrocellulose. The drying of the test device is performed in a vacuum cabinet to accelerate drying.

After this, the above-described required labels that are characteristic to each test are dosed to the predetermined label points 4. The drying is performed as described in the above-mentioned example.

### Example 6. A test device for myocardial infarct testing

An example that well describes the applications of the present invention is a test device for detecting myocardial infarct from a whole blood, plasma or serum sample of a patient.

The test device according to Figure 4 is produced for four different analytes. The test device produced as described in Example 1 comprises four identical channels that each further branch into two separate channels. The test device can thus be used to simultaneously determine from a single sample the presence of Troponin I, Myoglobin, Creatine kinase MB isoenzyme (CKMB) and C-reactive protein (CRP) in a patient sample.

As regards to the channels 1a and 1b of the test device, 0,1-0,5 µl of an antibody of Troponin I in a concentration of 1,0-5,0 mg/ml is applied into the channel 1a and 0,1-0,5 µl of an anti-mouse antibody in a concentration of 1,0-5,0 mg/ml into the channel 1b. The channel 1a forms a so-called test channel and the channel 1b forms a so-called internal functionality control channel.

The other reaction pair is formed of coloured particles coated with an antibody produced against Troponin I and applied as described in Example 1 to the predetermined label point 4 in the channel 1.

In addition to Troponin I specific tests for Myoglobulin, CKMB and C-reactive protein (CRP) are produced in the test device in the above-described manner.

All channels 1 in the test device are treated with a so-called blocking solution containing polyethylene glycol (PEG), TRITON-X-100 and trehalose (1,0-3,0 %) by dosing a sufficient amount of this to the sample application point 5 of the test device, from where it diffuses into each channel and fills the reactive zones of the nitrocellulose. The drying of the test device is performed in a vacuum cabinet to accelerate the drying.

After this, the labels characteristic to each test are applied to the predetermined label points 4 of the test device. The drying is performed as described in the above-mentioned examples.

The sample is applied in the middle of the test device, from where it diffuses radially to each identical channel initiating the test and control reactions if the patient sample contains an analyte corresponding to myocordial infarct markers. The analysis is performed either from a serum, plasma or whole blood sample, in which case a suitable filtering system removes the erythrocytes and leucocytes from the whole blood sample.

### Example 7. A test device for myocordial infarct testing

A test device according to Figure 11 is produced as described in Example 1 containing markings with information concerning specific tests for Troponin I and Myoglobulin.

The specific tests for Troponin I and Myoglobulin are produced as described in Example 6. Also the blocking of the channels and the dosing of the labels are performed according to Example 6. One of the channels acts as a control channel ensuring that the test has been correctly stored and that the reagents function.

### Example 8. A test device for respiratory infection testing

The following example describes the application of the present invention in respiratory infection cases, in which it is desired to measure class specific antibodies against a sought bacterium or virus antigen from serum, plasma or whole blood samples of a patient.

A test device according to Figure 6 is produced as described in Example 1. The antigen prepared from each analyzable bacterium or virus is placed in points 3 in test channels 1 in a concentration of 0,1-0,5 mg/ml, the total volume of each reagent being 0,1-0,5 µl. The nonreactive zones of the channels 1 are blocked and the test devices are dried as described in Example 1. Conjugates made of anti-IgG, anti-IgM or anti-IgA antibodies are placed in the label point 4 of the branch point of each test channel, respectively. They are dried as described above.

A patient sample (serum, plasma or whole blood) is applied to the sample application point 5 in the middle of the test device, from where it diffuses and transfers first to the conjugate points due to capillary action and reacts in the label points 4 in question with particle labels and subsequently migrates further towards test zones 3, where a reaction takes place if the sample contains the subclass specific antibody in question against the analyzable bacterium or virus. Positive results are detected in a similar manner as disclosed in the above examples.

### Example 9. A test device for cancer diagnostics

Example 9 describes the application of the present invention to a cancer diagnostical test device. The test device according to Figure 9 is produced as described in Example 1. The test device comprises five channels that are practically identical. 0,1-0,5 µl of monoclonal antibodies CA 125 (Cancer Antigen 125), PSA (prostate-specific antigen), pKAc (protein kinase A catalytic subunit), CEA (carcinoembryonic antigen), AFP (alphafetoprotein) produced against cancer markers are dosed in a concentration of 0,1-5,0 mg/ml for each test point in each channel (from left to right). After applying the antibodies, sufficient drying is performed, after which blocking is performed using polyvinyl alcohol (PVA), hexane sulphonic acid and sucrose. The test device is subsequently dried, after which it is ready for the dosing of the labels.

Respectively, a specific label made for the corresponding cancer marker, in which label gold particles are coated with a second antibody produced against the marker in question as described in earlier examples, is dosed in the label zone 4 in the beginning of each test channel. After the dosage, the test device is dried and packed in a protecting plastic casing.

10 µl of a patient sample (serum, plasma, whole blood, etc.) is applied to the sample application point 5, from where it migrates into channels 1 corresponding to each cancer marker, dissolving the label from the label point 4 and further to the test zones 3. Provided that the analyzable cancer marker is present in the sample, a visible test result emerges in the channel in question.

### Example 10. A test device for virus antigen detection

Example 10 describes the application of the present invention in a test device for detection of virus antigens.

To detect the Rota virus, the test device shown in Figure 10, in which the channelization is made as described in Example 1, is produced by placing in the first channel 1 a blot made of a specific antibody produced against the virus antigen into the test zone 3 which is an antibody against the label antibody used, of the label point 4.

The third channel 1 of the same test device is used to detect an Adeno virus antigen by placing in that channel a line or blot to the test zone 3 comprising a specific antibody against the label antibody in question placed in the label zone 4.

The second channel of the test device is used as a control channel to test the functionality of the test device. A non-specific conjugate is added into the label point and the anti-mouse antibody is added to the test zone.

The reactive points of channels 1 are blocked and the test devices are dried as described in Example 1. Labels characteristic to each assay are dosed to the label point 4 of the branch point of each test channel. They are dried as described above.

A patient sample (diluted faecal sample) is applied to the sample application point 5 of the test device, from where it diffuses and migrates first into the conjugate points due to capillary action and reacts with the particle labels in the label points 4 in question and subsequently continues to the test zones 3, where a reaction takes place if the sample contains the analyzable virus antigen. The positive results are detected in a similar manner as disclosed in the above examples.

## Claims

1. An immunodiffusion-based test device comprising a porous carrier material, wherein a specific binding reagent (immunoreagent) is applied in the form of a zone or a blot, a detectable label, whereto a second specific binding reagent is coupled, and which is supplied separately or pre-applied to the porous carrier, is mobilizable by the sample, and a sample application site optionally provided with a filter, **characterized in that** it comprises in the porous carrier material a network of channels and markings with information concerning the test, which are formed by etching porous carrier material with laser treatment, which network of channels comprises two or more channels (1), separated by a treated area (2), one or more specific binding reagents (3) immobilized in them, an optional label site (4) placed in the channel near the sample application site (5), or in the sample application site (5) itself, which is placed in a manner that enables an even distribution of the sample into each channel.

2. The test device according to claim 1, **characterized in that** it enables making a diagnosis based on a simultaneously or parallelly performed recognition of one or more several syndrome-producing agents or analytes.

3. The test device according to claim 1, **characterized in that** it is used to simultaneously recognize more than one allergy-producing agents, myocardial infarction markers, venereal disease-producing agents, blood screening analytes, respiratory infection-producing agents, other infectious disease-producing agents and/or cancer markers.

4. The test device according to claim 1, **characterized in that** the porous carrier is nitrocellulose.

5. The test device according to claim 1, **characterized in that** the specific binding reagents are antibodies, antibody fragments, recombinant antibodies, recombinant antibody fragments, antigens, lectins, receptors and/or ligands.

6. The test device according to claim 1, **characterized in that** the labeled reagents are latex, gold, metal or colouring agent particles, or fluorescent substances.

7. A method for producing a test device according to claim 1, wherein the specific recognizing immunoreagent is immobilized in a porous material, the porous material is treated with a substance rendering it inert, and wherein a sample application point is optionally provided with a filter and a detectable label to which a specific binding reagent is bound, **characterized in that**
(a) the porous material is etched with laser to form simultaneously on the porous material
(i) a channel network of a desired shape and size, the network comprising two or more channels (1) and a treated area (2) and
(ii) markings with information concerning the test;
(b) one or more recognizing specific binding reagents are immobilized in each channel (1) as a zone or blot (3).

8. The method according to claim 7, **characterized in that** the substance by which the porous material is rendered inert is a mixture comprising natural or synthetic polymers, such as albumin and casein or PEG (polyethylene glycol) and PVA (polyvinyl alcohol), nonionic detergents such as hexane sulphonic acid and TRITON-X-100, BRIJ, and preservatives such as sugar, for example sucrose and trehalose, or their derivatives.

9. The method according to claim 7, **characterized in that** the test device is dried to a relative humidity of not over 8 % and hermetically packed after application of the reagents.

10. Use of a test device according to claim 1 for simultaneous or parallel performing of several assays for diagnosing a sought disease and/or syndrome, **characterized in that** the sample is applied to an application point (5) of the test device containing a label mobilizable by the analyzable sample and a second specific binding reagent coupled to it, from which point the sample and the reagents migrate evenly into the channels of the channel network formed by a laser treatment, where they either react or do not react with the specific binding reagent in its immobilization point (3), from where the positive or negative results are directly readable.

11. Use of a test device according to claim 1 for simultaneous or parallel performing of several assays for recognition of a sought disease and/or syndrome, **characterized in that** the sample is applied to an application point (5) of the test device, from which it migrates into a channel where it is mixed to and reacts with the label mobilizable by the sample and the second specific binding reagent bound to it, after which the sample and reagents migrate evenly into the channels of the channel network formed by a laser treatment, where they either react or do not react with the specific binding reagent in its immobilization point (3), from where the positive or negative results are directly readable.

12. Use of a test device according to claim 1 for simultaneous or parallel performing of several assays for recognition of a sought disease and/or syndrome, **characterized in that** the sample is mixed to a separate label and a second specific binding reagent coupled to it and the mixture is applied to an application point (5) of the test device, from which the sample and the reagents migrate evenly into the channels of the channel network formed by a laser treatment, where they either react or do not react with the specific binding reagent in its immobilization point (3), from where the positive or negative results are directly readable.

## Patentansprüche

1. Eine auf Immundiffusion basierende Testvorrichtung, umfassend ein poröses Trägermaterial, worin ein spezifisches Bindungsreagens (Immunreagens) in Form einer Zone oder eines Blots aufgebracht wird, eine nachweisbare Markierung, woran ein zweites spezifisches Bindungsreagens gekoppelt wird und das getrennt zugeführt wird oder auf dem porösen Träger vorher aufgebracht wird, durch die Probe mobilisierbar ist, und eine Probenaufbringstelle, die optional mit einem Filter versehen ist, **dadurch gekennzeichnet, dass** sie in dem porösen Trägermaterial ein Netzwerk von Kanälen und Kennzeichen mit Informationen betreffend den Text umfasst, die durch Ätzen des porösen Trägermaterials mit Laserbehandlung gebildet werden, wobei das Netzwerk von Kanälen zwei oder mehr Kanäle (1) umfasst, getrennt durch einen behandelten Bereich (2), ein oder mehr spezifische Bindungsreagentien (3), die darin immobilisiert sind, eine optionale Markierungsstelle (4), platziert in dem Kanal nahe der Probenaufbringstelle (5) oder in der Probenaufbringstelle (5) selbst, die auf eine Weise platziert ist, dass eine gleichmäßige Verteilung der Probe in jeden Kanal ermöglicht wird.

2. Testvorrichtung gemäß Anspruch 1, **dadurch gekennzeichnet, dass** eine Diagnose ermöglicht wird, basierend auf einer simultan oder parallel durchgeführten Erkennung von einem oder mehr etliche Syndrome-erzeugenden Mitteln oder Analyten.

3. Testvorrichtung gemäß Anspruch 1, **dadurch gekennzeichnet, dass** sie verwendet wird, um simultan mehr als ein Allergie erzeugendes Mittel, Myoacard-Infarktmarker, Geschlechtskrankheiten erzeugende Mittel, Blutscreening-Analyten, respiratorische Infektionen erzeugende Mittel, andere Infektionserkrankungen erzeugende Mittel und/oder Krebsmarker zu erkennen.

4. Testvorrichtung gemäß Anspruch 1, **dadurch gekennzeichnet, dass** der poröse Träger Nitrocellulose ist.

5. Testvorrichtung gemäß Anspruch 1, **dadurch gekennzeichnet, dass** die spezifischen Bindungsreagentien Antikörper, Antikörperfragmente, rekombinante Antikörper, rekombinante Antikörperfragmente, Antigene, Lectine, Rezeptoren und/oder Liganden sind.

6. Testvorrichtung gemäß Anspruch 1, **dadurch gekennzeichnet, dass** die markierten Reagentien Latex, Gold, Metall oder Färbemittelpartikel oder fluoreszierende Substanzen sind.

7. Verfahren zur Erzeugung einer Testvorrichtung gemäß Anspruch 1, wobei das spezifische erkennende Immunreagens in einem porösen Material immobilisiert ist, wobei das poröse Material mit einer Substanz behandelt wird, die es inert macht, und wobei ein Probenaufbringpunkt optional mit einem Filter und einer nachweisbaren Markierung versehen ist, woran ein spezifisches Bindungsreagens gebunden ist, **dadurch gekennzeichnet, dass**
(a) das poröse Material mit Laser geätzt ist, um simultan auf dem porösen Material
(i) ein Kanalnetzwerk mit gewünschter Form und Größe zu bilden, wobei das Netzwerk zwei oder mehr Kanäle (1) und einen behandelten Bereich (2) umfasst, und
(ii) Kennzeichen mit Informationen, die den Test betreffen, zu bilden;
(b) ein oder mehr erkennende spezifische Bindungsreagentien in jedem Kanal (1) als Zone oder Blot (3) immobilisiert sind.

8. Verfahren gemäß Anspruch 7, **dadurch gekennzeichnet, dass** die Substanz, durch die das poröse Material inert gemacht wird, eine Mischung ist, umfassend natürliche oder synthetische Polymere wie z.B. Albumin und Casein oder PEG (Polyethylenglycol) und PVA (Polyvinylalkohol), nicht-ionische Detergentien wie z.B. Hexansulphonsäure und Triton-X-100, BRIJ und Konservierungsmittel wie z.B. Zucker, beispielsweise Saccharose und Trehalose oder ihre Derivate.

9. Verfahren gemäß Anspruch 7, **dadurch gekennzeichnet, dass** die Testvorrichtung nach Aufbringen der Reagentien auf eine relative Feuchtigkeit von nicht über 8% getrocknet und hermetisch verpackt wird.

10. Verwendung einer Testvorrichtung gemäß Anspruch 1 für das simultane oder parallele Durchführen etlicher Assays für eine Diagnose einer gesuchten Erkrankung und/oder eines Syndroms, **dadurch gekennzeichnet, dass** die Probe auf einen Aufbringpunkt (5) der Testvorrichtung aufgebracht wird, enthaltend eine Markierung, die durch die analysierbare Probe mobilisierbar ist, und ein zweites spezifisches Bindungsreagens daran gekoppelt ist, von welchem Punkt die Probe und die Reagentien gleichmäßig in die Kanäle des Kanalnetzwerks migrieren, das durch eine Laserbehandlung gebildet wird, wodurch sie entweder mit dem spezifischen Bindungsreagens an seinem Immobilisierungspunkt (3) reagieren oder nicht, von wo aus die positiven oder negativen Ergebnisse direkt ablesbar sind.

11. Verwendung einer Testvorrichtung gemäß Anspruch 1 für das simultane oder parallele Durchführen etlicher Assays zur Erkennung einer gesuchten Erkrankung und/oder eines Syndroms, **dadurch gekennzeichnet, dass** die Probe auf einen Aufbringpunkt (5) der Testvorrichtung aufgebracht wird, von wo sie in einen Kanal migriert, wo sie mit der Markierung vermischt wird und reagiert, die durch die Probe mobilisierbar ist, und das zweite spezifische Bindungsreagens daran gebunden ist, woraufhin die Probe und die Reagentien gleichmäßig in die Kanäle des Kanalnetzwerks migrieren, das durch eine Laserbehandlung gebildet wird, wo sie mit dem spezifischen Bindungsreagens an seinem Immobilisierungspunkt (3) entweder reagieren oder nicht, von wo aus die positiven oder negativen Ergebnisse direkt ablesbar sind.

12. Verwendung einer Testvorrichtung gemäß Anspruch 1 für das simultane oder parallele Durchführen etlicher Assays zur Erkennung einer gesuchten Erkrankung und/oder eines Syndroms, **dadurch gekennzeichnet, dass** die Probe mit einer getrennten Markierung und einem daran gebundenen zweiten spezifischen Bindungsreagens vermischt wird und dass die Mischung auf einen Aufbringpunkt (5) der Testvorrichtung aufgebracht wird, von wo aus die Probe und die Reagentien gleichmäßig in die Kanäle des Kanalnetzwerkes migrieren, das durch eine Laserbehandlung gebildet wird, wo sie mit dem spezifischen Bindungsreagens an seinem Immobilisierungspunkt (3) entweder reagieren oder nicht, von wo aus die positiven oder negativen Ergebnisse direkt ablesbar sind.

## Revendications

1. Dispositif d'analyse à base d'immunodiffusion comprenant un matériau porteur poreux, dans lequel un réactif de fixation spécifique (immunoréactif) est appliqué sous forme d'une zone ou d'une tache, un marqueur détectable, auquel un second réactif de fixation spécifique est couplé, et qui est fourni séparément ou qui est appliqué préalablement sur le porteur poreux, est mobilisable par l'échantillon, et un site d'application de l'échantillon éventuellement pourvu d'un filtre, **caractérisé en ce qu'**il comprend dans le matériau porteur poreux un réseau de canaux et des marquages comportant des informations concernant le test, qui sont formés en gravant le matériau porteur poreux par un traitement laser, lequel réseau de canaux comprend deux ou plusieurs canaux (1), séparés par une zone traitée (2), un ou plusieurs réactifs de fixation spécifique (3) immobilisés dans les canaux, un site de marqueur facultatif (4) placé dans le réseau près du site d'application de l'échantillon (5), ou dans le site même d'application de l'échantillon (5), qui est situé de manière à permettre une répartition égale de l'échantillon dans chaque canal.

2. Dispositif d'analyse selon la revendication 1, **caractérisé en ce qu'**il permet d'établir un diagnostic s'appuyant sur une reconnaissance simultanée ou parallèle d'un ou plusieurs agents ou analytes responsables d'un syndrome.

3. Dispositif d'analyse selon la revendication 1, **caractérisé en ce qu'**il est utilisé pour reconnaître simultanément plusieurs agents allergènes, marqueurs d'infarctus du myocarde, agents responsables de maladies vénériennes, analytes de dépistage hématologique, agents responsables d'infections respiratoires, agents responsables d'autres infections et/ou marqueurs de cancer.

4. Dispositif d'analyse selon la revendication 1, **caractérisé en ce que** le porteur poreux est la nitrocellulose.

5. Dispositif d'analyse selon la revendication 1, **caractérisé en ce que** les réactifs de fixation spécifique sont des anticorps, des fragments d'anticorps, des anticorps recombinés, des fragments d'anticorps recombinés, des antigènes, des lectines, des récepteurs et/ou des ligands.

6. Dispositif d'analyse selon la revendication 1, **caractérisé en ce que** les réactifs marqués sont des particules de latex, d'or, de métal ou d'agent colorant, ou des substances fluorescentes.

7. Procédé de production d'un dispositif d'analyse selon la revendication 1, dans lequel l'immunoréactif de reconnaissance spécifique est immobilisé dans un matériau poreux, le matériau poreux est traité avec une substance le rendant inerte, et dans lequel un point d'application de l'échantillon est éventuellement pourvu d'un filtre et d'un marqueur détectable auquel un réactif de fixation spécifique est fixé, **caractérisé en ce que** :
(a) le matériau poreux est gravé au laser pour former simultanément sur le matériau poreux
(i) un réseau de canaux d'une forme et d'une dimension souhaitées, le réseau comprenant deux canaux (1) ou plus et une zone traitée (2) et
(ii) des marquages comportant des informations concernant le test ;
(b) un ou plusieurs réactifs de fixation spécifique de reconnaissance sont immobilisés dans chaque canal (1) sous forme d'une zone ou d'une tache (3).

8. Procédé selon la revendication 7, **caractérisé en ce que** la substance par laquelle le matériau poreux est rendu inerte est un mélange comprenant des polymères naturels ou synthétiques, comme l'albumine et la caséine ou le PEG (polyéthylèneglycol) et le PVA (alcool polyvinylique), des détergents non ioniques comme l'acide hexane sulfonique et le Triton-X-100, le Brij, et des conservateurs comme le sucre, par exemple le saccharose et le tréhalose, ou leurs dérivés.

9. Procédé selon la revendication 7, **caractérisé en ce que** le dispositif d'analyse est séché à une humidité relative ne dépassant pas 8 % et est hermétiquement emballé après application des réactifs.

10. Utilisation d'un dispositif d'analyse selon la revendication 1 pour exécuter simultanément ou en parallèle plusieurs dosages en vue de diagnostiquer une maladie et/ou un syndrome recherché(e), **caractérisé en ce que** l'échantillon est appliqué sur un point d'application (5) du dispositif d'analyse contenant un marqueur mobilisable par l'échantillon analysable et un second réactif de fixation spécifique couplé à celui-ci, à partir duquel point l'échantillon et les réactifs migrent de façon égale dans les canaux du réseau de canaux formé par un traitement laser, où ils réagissent ou non avec le réactif de fixation spécifique dans son point d'immobilisation (3), à partir duquel les résultats positifs ou négatifs sont directement lisibles.

11. Utilisation d'un dispositif d'analyse selon la revendication 1 pour exécuter simultanément ou en parallèle plusieurs dosages en vue de reconnaître une maladie et/ou un syndrome recherché(e), **caractérisé en ce que** l'échantillon est appliqué sur un point d'application (5) du dispositif d'analyse, à partir duquel il migre dans un canal où il est mélangé et où il réagit avec le marqueur mobilisable par l'échantillon et le second réactif de fixation spécifique fixé à celui-ci, après quoi l'échantillon et les réactifs migrent de façon égale dans les canaux du réseau de canaux formé par traitement laser, où ils réagissent ou non avec le réactif de fixation spécifique dans son point d'immobilisation (3), à partir duquel les résultats positifs ou négatifs sont directement lisibles.

12. Utilisation d'un dispositif d'analyse selon la revendication 1 pour exécuter simultanément ou en parallèle plusieurs dosages en vue de reconnaître une maladie et/ou un syndrome recherché(e), **caractérisé en ce que** l'échantillon est mélangé à un marqueur séparé et un second réactif de fixation spécifique couplé à celui-ci et le mélange est appliqué sur un point d'application (5) du dispositif d'analyse, à partir duquel l'échantillon et les réactifs migrent de façon égale dans les canaux du réseau de canaux formé par traitement laser, où ils réagissent ou non avec le réactif de fixation spécifique dans son point d'immobilisation (3), à partir duquel les résultats positifs ou négatifs sont directement lisibles.
